# EUROPEAN PATENT APPLICATION

(11) **EP 2 556 836 A1**
(43) Date of publication of application: **13.02.2013**
(21) Application number: 10735172.8
(22) Date of filing: 15.04.2010
(51) Int. Cl.: A61K 36/815, A61K 36/734, A61K 36/67, A61K 36/575, A61K 36/539, A61K 36/53, A61K 36/21, A61K 36/185, A61P 1/04, A61K 125/00, A61K 129/00, A61K 131/00

(54) **TRADITIONAL CHINESE MEDICINE COMPOSITION FOR TREATING GASTRIC ULCER AND PREPARATION METHOD THEREOF**

(30) Priority: 09.04.2010 CN 201010143024
(71) Applicant: Tongling Weixin Investment & Consulting Co., Ltd, Tongling, Anhui 244000 (CN)
(72) Inventor: ZHANG, Baoyu, Anhui 244000 (CN)
(74) Representative: Larcher, Dominique
(86) International application number: PCT/CN2010/000508
(87) International publication number: WO 2011/123997

(57) **Abstract**

The present invention relates to a herbal composition for treating gastric ulcer, a method for preparing said herbal composition.

## Description

### Technical Field

The present invention relates to a novel herbal composition for the treating gastric ulcer. More particularly, the present invention relates to a novel herbal composition which is effective against pyloric ligation induced ulcer model and histamine induced ulcer model. The present invention also relates to a method for the preparation of the composition.

### Background Art

Various theories have been proposed with respect to a cause of ulcer in human. In particular, it has been elucidated that stress, taking of non-steroidal anti-inflammatory drugs for curing rheumatic diseases, and the like are closely related to ulcer formation, mainly due to relatively excess gastric or duodenal acid secretion. Accordingly it is important to suppress the acid secretion in order to prevent ulcer formation and to cure it.

On the other hand it has been considered that Helicobacter pylori, which is a rod normally existing in stomach, generates ammonia due to its strong urease activity, thereby inducing ulcer. Since it persistently lives within mucus and mucosa, it becomes the greatest cause for recurrence of ulcer. Accordingly, it has been considered that the recurrence of ulcer can be prevented if this bacterium is sterilized

The composition of the present invention should not be treated as an obvious one as none of the citations are able to provide all the advantages of the present invention..

### Summary of the Invention

The main object of the present invention is to provide a novel herbal composition for treating gastric ulcer.

Yet another object of the present invention is to provide a process for the preparation of the composition.

The present invention provides a novel herbal composition for treating gastric ulcer. More particularly, the present invention relates to a novel herbal composition which is effective against pyloric ligation induced ulcer model and histamine induced ulcer model. Also, the present invention provides a process for the preparation of the composition.

### Detailed Description of the present Invention

In accordance with the first object of the present invention, there is provided a herbal composition for treating gastric ulcer, the said composition is consisting of 4-10% by wt. of an extract from Radix Scutellariae and 4-11% by wt. of an extract from Radix Lamiophlomidis and optionally comprises 5-8 % by wt. of an extract from Radix Achyranthis, 5-11 % by wt. of an extract from Fructus Crataegi, 5- 9% by wt. of an extract from Fructus Lycii, 8-11 % by wt. of an extract from Punica grantum, 4-9% by wt. of an extract from Ziniber officnale, 2-11 % by wt. of an extract from Fructus Ribis manschurici, 8-12% by wt. of an extract from Piper longue and 2- 11 % by wt. of an extract from Coxtex Magnoliae along with one or more pharmaceutically acceptable additives/carriers.

In an embodiment of the present invention, the extract is an aqueous extract.

In another embodiment of the present invention, the plant part of Radix Scutellariae, Radix Lamiophlomidis and Radix Achyranthis is root.

In still another embodiment of the present invention, the plant part of Vitis vinifera, Fructus Lycii, Fructus Ribis manschurici and Piper longum is fruit.

In yet another embodiment of the present invention, the plant part of Pashica grantum is fruit rind.

In one more embodiment of the present invention, the plant part of Ziniber officnale is rhizome.

In one another embodiment of the present invention, the plant part of Coxtex Magnoliae is bark.

In accordance with the second object of the present invention, there is provided a process for the preparation of the novel herbal composition for treating gastric ulcer, the said process comprises getting all the chinese herbes, adding solvent to extract them, and mixing the extracts with one or more pharmaceutically acceptable additives/carriers.

In another embodiment of the present invention, the extract is an aqueous extract.

### Brief Description of the Tables

In the tables accompanying the specification,
Table 1 represents the effect of Omeprazole (a standard drug) and the new herbal composition (HF) against Cold Restraint Ulcer (CRU) Model.
Table 2 compares the percentage protection of Omeprazole and the Herbal composition (HF) against Cold Restraint Ulcer (CRU) Model.
Table 3 gives the effect of Omeprazole and the Herbal composition (HF) against Aspirin induced ulcer model.
Table 4 gives the percentage protection of Omeprazole and the Herbal composition (HF) against aspirin induced ulcer model.
Table 5 gives the effect of Omeprazole and the Herbal composition (HF) against histamine induced duodenal ulcer in Guinea pig.
Table 6 gives the percentage protection of Omeprazole and the Herbal composition (HF) against histamine induced duodenal ulcer in Guinea pigs.
Table 7 gives the effect of Omeprazole and the Herbal composition (HF) against Ethanol Induced Ulcer Model.
Table 8 gives the percentage protection of Omeprazole and the Herbal composition (HF) against Alcohol induced Ulcer Model.
Table 9 gives the effect of Omeprazole and the Herbal composition (HF) against pyloric ligation induced Ulcer.
Table 10 gives the percentage protection of Omeprazole and the Herbal composition (HF) against pyloric ligation induced Ulcer.
Table 11 gives the composition of the Herbal composition (HF) of the present invention.

The present invention is further described with reference to the following experiments which are given by way of illustration and therefore should not be construed to limit the scope of the invention in any manner.

Experimental protocol: Invivo experiments: The Applicants have carried out several experiments under different induced ulcer conditions and the effect of the herbal composition were studied and are tabulated herebelow. The effect of the herbal composition has been compared with respect to a known anti-ulcer drug"Omeprazole".

### EXPERIMENT 1: EFFECT ON COLD RESTRAINT ULCERS (CRU) MODEL METHOD:

Adult rats of either sex, weighing 150-175 grams are fasted for 24 hours in metallic cages with raised mesh bottoms to prevent coprophagia and were allowed free access to water. The test drugs were administered 45 minutes before immobilizing the animals. The rats were immobilized in the restraint cage and kept at 4°C in BOD incubator for 2 hours (According to the method of Senay and Levine 1967). The animals were sacrificed immediately after the restraint period. The abdomen was cut opened; stomach was taken out and incised along the greater curvature to observe the gastric lesions with the help of Magnascope (5X magnification) The following arbitrary scoring system was used to grade the severity and intensity of the lesions: 1. Shedding of epithelium = 10 2. Petechial and frank hemorrhages = 20 3. One or two ulcers = 30 4. More than two ulcers = 40 5. Perforated ulcers = 50 The presence of any of these lesions was considered as a positive ulcerogenic response which has been shown as percentage of rats showing gastric lesions. The severity of ulcers is expressed in terms of ulcer index, which is the mean score of gastric lesions of all the rats in a group.

The term Ulcer Index is defined as: Ulcer Index (U. I.) = Us + Up x 10-1 where Us = Mean severity of ulcer score and Up = Percentage of animals with Ulcer incidences The percentage protection is calculated as follows: Percentage protection = (C-T/C) x 100. where C= Number of animals showing ulcer response in control group and T= Number of animals showing ulcer response in test group.

The effect of the herbal composition of the present invention hereafter referred to as"HF" against Cold Restraint Ulcer Model (CRU) is given in Table 1. The effect of the standard drug"Omeprazole"is also given in Table 1 given at the end of the description.

Percentage protection of the herbal composition of the present invention (HF) and Omeprazole against CRU model are tabulated in Table 2 given at the end of the description. INFERENCE: The composition of the present invention is significantly effective in CRU model.

### EXPERIMENT 2: EFFECT ON ASPIRIN INDUCED GASTRIC ULCER MODEL Method:

Gastric ulceration was induced by aspirin according to the method of Djahanguiri (1969). Aspirin (150 mg/Kg.) was administered per orally as a suspension in gum-acacia and the animal was sacrificed 5 hr. after the aspirin treatment and the ulcer index with protection index were calculated.

The effect of HF against aspirin induced gastric ulcer is given in Table 3. The effect of the standard drug"Omeprazole"is also given in Table 3 given at the end of the description. Percentage protection of the herbal composition of the present invention (HF) and Omeprazole against this model are tabulated in Table 4 given at the end of the description. INFERENCE: The herbal composition of the present invention is effective against Aspirin induced gastric ulcer model.

### EXPERIMENT 3: EFFECT ON HISTAMINE INDUCED ULCER MODEL Method: 1.

Animals were fasted for 24 hours with access to water.
2. The drug was given orally 1 hour prior to the histamine administration.
3. Histamine was administered in a dose of 0.25 mg/Kg, i. m. at 30 minutes interval for 7 times and it induced 100 % duodenal ulceration in guinea pig (According to the method of Watt and Eagleton 1964).
4. The animals were sacrificed after half an hour of last injection under ether anesthesia.
5. The stomach along with duodenum were removed washed thoroughly and examined for the lesions. Ulcer index and protection index were calculated.

The effect of HF against Histamine induced duodenal ulcer is given in Table 5. The effect of the standard drug"Omeprazole"is also given in Table 5 given at the end of the description. Percentage protection of the herbal composition of the present invention (HF) and Omeprazole against Histamine induced duodenal ulcer are tabulated in Table 6 given at the end of the description.

### INFERENCE: The Herbal composition of the present invention"HF"shows significant anti ulcer effect against this model.

### EXPERIMENT 4: EFFECT ON ALCOHOL INDUCED GASTRIC ULCERS IN RATS

### Method: 1.

Adult rats of either sex were taken ; weighing 150-175 grams were fasted for 24 hours with free access to water.
2. The test drugs were administered (p. o. ) 45 minutes before alcohol administration.
3.1 ml of chilled absolute alcohol was administered (p. o. ) to the rats (According to the Wittetal).
4. Immediately after 1 hour, the animals were anesthetized, abdomen was cut opened stomach was taken out and incised along the greater curvature to observe the gastric lesions.

The ulcers are examined under the 5X magnification with the help of magnascope.

Absolute ethanol lesions appears as blackish lesions grouped in patches of varying size, usually parallel to the major axis of the stomach. 5. The lengths of the lesions are measured and summated to give a total lesion score, then calculated and expressed in percentage.

The effect of HF against alcohol induced ulcer model is given in Table7. The effect of the standard drug"Omeprazole"is also given in Table 7 given at the end of the description. Percentage protection of the herbal composition of the present invention (HF) and Omeprazole against alcohol induced ulcer model are tabulated in Table 8 given at the end of the description.

### INFERENCE: The composition of the present invention does not show any significant effect against alcohol induced gastric ulcer model.

### EXPERIMENT 5: EFFECT ON PYLORIC LIGATION INDUCED ULCER MODEL

### Method: 1.

Animals were fasted for 24 hours in the raised mesh bottom cages to prevent coprophagia and were allowed free access to water.
2. The control group of rats was feed with the vehicle and the experimental groups with their respective drugs 45 minutes prior to the ligation.
3. The animal was anesthetized, abdomen was cut opened under xiphoid process, and the pyloric portion of the stomach was slightly lifted and ligated avoiding any damage to the adjacent blood vessels (According to the method of Shay et al. 1945).
4. The animals were stitched and kept for 4 hours with free access to water.
5. After 4 hours the animals were sacrificed under ether anesthesia and the stomach was dissected out incised along the greater curvature.
6. The stomach was washed thoroughly and the ulcer index was scored as per in other ulcer models.

The effect of HF against pyloric ligation induced ulcer is given in Table 9. The effect of the standard drug"Omeprazole"is also given in Table 9 given at the end of the description. Percentage protection of the herbal composition of the present invention (HF) and Omeprazole against pyloric ligation induced ulcer are tabulated in Table 10 given at the end of the description.

### INFERENCE: On comparison, Herbal composition shows high anti ulcer activity. The anti ulcer activity of the herbal composition is higher than that of Omeprazole.

### EXPERIMENT 6: HERBS AND PREPARATION OF THE COMPOSITION Method: For

the purpose of conducting animal experiment all the herbs are washed dried and pulverized. All the herbs are taken in the proportion as shown in Table 11. To this water was added and boiled and concentrated to appropriate consistency. The components and their proportions of the standard"herbal composition" (HF) according to one embodiment of the present invention are listed in Tablell given at the end of the description. The parts of the herbs which can be used is also mentioned. The placebo preparation is designed to taste, smell and look like an Ayurvedic herbal formulation.

**TABLE 1: Effect of New herbal composition (HF) against Cold Restraint Ulcer Model (CRU) with Omeprazole as a standard drug.**

| Compound and Doses | **Ulcer severity_**(type of lesions) **Scores** (No. of rats showing lesions / No. of rats tested) | | | | | Mean severity of ulcer score | Percentage of ulcer incidence (No. of rats showing ulcer/ total No. of rats used) | Ulcer index | Protection index |
|---|---|---|---|---|---|---|---|---|---|
| | 10 | 20 | 30 | 40 | 50 | | | | |
| Control CRU | - | 6/10 | 4/10 | -- | -- | 24 | 100 (10/10) | 12.4 | 00 |
| | | | | | | | | | |
| CRU+HF (50mg/Kg, p..o.) | 4/10 | 2/10 | -- | -- | -- | 08 | 60 (6/10) | 6.8 | 45.16 |
| CRU+Ome prazole (10 mg/Kg, p.o.) | 3/10 | 2/10 | -- | -- | -- | 07 | 50 (5/10) | 5.7 | 54.03 |

| | | | | | | | | | |
|---|---|---|---|---|---|---|---|---|---|
| In the table, 10 = Shedding of epithelium; 20 = Petechial and frank hemorrhages; 30 = One or two ulcers; 40 = More than two ulcers; 50 = Perforated ulcers. | | | | | | | | | |

**TABLE 2: Percentage protection of Herbal composition against Cold Restraint Ulcer Model (CRU) taking Omeprazole as a standard drug.**

| **GROUP** | **PERCENTAGE PROTECTION** |
|---|---|
| **Herbal composition (HF)** of the present invention (50 mg/Kg) | 83.58 |
| ***Omerprazole** (10 mg/Kg) | 100% |

| | |
|---|---|
| *The protection of Omeprazole was taken as 100% as it was the standard compound and the percentage protections of other compounds are in respect to the protection of Omeprazole. | |

**TABLE 3: Effect of Herbal composition against Aspirin induced ulcer model with Omeprazole as a standard drug.**

| Compound and Doses | Ulcer severity (type of lesions) Scores (No. of rats showing lesions / No. of rats tested) | | | | | Mean severity of ulcer score | Percentage of ulcer incidence (No. of rats showing ulcer/total No. of rats used) | Ulcer index | Prote ction index |
|---|---|---|---|---|---|---|---|---|---|
| | 10 | 20 | 30 | 40 | 50 | | | | |
| Control Aspirin (150 mg/Kg, p.o.) | - | 2/6 | 4/6 | -- | -- | 26.6 | 100 (6/6) | 12.66 | 00 |
| Aspirin + Herbal composition 100mg/Kg) | 2/6 | 3/6 | -- | -- | -- | 13.3 | 83.3 (5/6) | 9.66 | 23.6 9 |
| Aspirin + Omeprazole (20 mg/Kg) | 2/6 | 1/6 | -- | -- | -- | 6.6 | 50 (3/6) | 5.66 | 54.8 1 |

| | | | | | | | | | |
|---|---|---|---|---|---|---|---|---|---|
| In the table, 10 = Shedding of epithelium; 20 = Petechial and frank hemorrhages; 30 = One or two ulcers; 40 = More than two ulcers; 50 = Perforated ulcers. | | | | | | | | | |

**TABLE 4: Percentage protection of Herbal composition against aspirin induced ulcer model taking Omeprazole as a standard drug.**

| **Compound** | **Percentage protection** |
|---|---|
| Herbal composition (50 mg/Kg) | 60.12 |
| * Omeprazole (10 mg/Kg) | 100 |

| | |
|---|---|
| * The protection of Omeprazole was taken as 100% as it was the standard compound and the percentage protections of other compounds are in respect to the protection of Omeprazole. | |

**TABLE 5: Effect of Herbal composition against histamine induced duodenal ulcer in Guinea pig with Omeprazole as a standard drug.**

| Groups and doses of compou nds | Ulcer severity (type of lesions) Scores (No. Guinea pig showing lesions / No. Guinea pig tested) | | | | | Mean severity of ulcer score | Percentage of ulcer incidence (No. of animals showing ulcer/total No. of animals used) | Ulcer index | % Prote ction | Volume of gastric fluid (mL) |
|---|---|---|---|---|---|---|---|---|---|---|
| | 10 | 20 | 30 | 40 | 50 | | | | | |
| Ulcer Control (Histam ine (25 mg/Kg, i.m.) | -- | -- | 1/3 | 2/3 | -- | 36.6 | 100 (3/3) | 13.66 | 00 | 4.33±1.1 |
| Histami ne+HF (50 mg/Kg., p.o.) | -- | -- | -- | 1/3 | -- | 13.33 | 33.3(1/3) | 4.66 | 65.88 | 2.7±0.37 |
| Histami ne + Omepra zole (10 mg/Kg, p.o.) | -- | 1/3 | -- | -- | -- | 6.66 | 33.33 (1/3) | 3.99 | 70.79 | 1.0±0.2 |

**TABLE 6: Percentage protection of Herbal composition against histamine induced duodenal ulcer in Guinea pig taking Omeprazole as a standard drug.**

| **COMPOUND** | **PERCENTAGE PROTECTION** |
|---|---|
| Herbal composition | 93.07 |
| * Omeprazole | 100 |

| | |
|---|---|
| * The protection of Omeprazole was taken as 100 % as it was the standard compound and the percentage protections of other compounds are in respect to the protection of Omeprazole. | |

**TABLE 7: Effect of Herbal composition against Ethanol Induced Ulcer Model with Omeprazole as a standard drug.**

| **COMPOUND** | **LENGTH OF HEMORRHAGIC BANDS (mm ± SE)** |
|---|---|
| Ethanol Control | 73.5±1.5 |
| Herbal composition (HF) of the present invention (100mg/Kg,p.o.) + Ethanol | 69.0±7.0 |
| Omeprazole (100mg/Kg,p.o.) + Ethanol | 56.0±9.12 |

**TABLE 8: Percentage protection of Herbal composition against Alcohol induced Ulcer Model taking Omeprazole as a standard drug.**

| **COMPOUNDS** | **PROTECTION** |
|---|---|
| Herbal composition | 25.71 |
| * Omeprazole | 100.0 |

| | |
|---|---|
| * The protection of omeprazole was taken as 100 % as it was the standard compound and the percentage protections of other compounds are in respect to the protection of omeprazole. | |

**TABLE 9: Effect of Herbal composition against pyloric ligation induced Ulcer taking Omeprazole as a Standard drug**

| Groups | Ulcer Index | Protection Index |
|---|---|---|
| Ligation Control | 16.6 | 00 |
| Ligation + Herbal composition (50 mg/Kg, p.o.) | 4.2 | 65.89 |
| Ligation + Omeprazole (10 mg/Kg, p.o.) | 6.6 | 51.44 |

**TABLE 10: Percentage protection of Herbal composition against pyloric ligation induced Ulcer Model taking Omeprazole as a standard drug.**

| **COMPOUNDS** | **PROTECTION** |
|---|---|
| Herbal composition | 124.0 |
| * Omeprazole | 100 |

| | |
|---|---|
| * The protection of Omeprazole was taken as 100 % as it was the standard compound and the percentage protections of other compounds are in respect to the protection of Omeprazole. | |

**TABLE11: Composition of the Herbal composition (HF) of the present invention**

| **S.NO** | **NAME OF THE INGREDIENT (PART USED)** | **%** |
|---|---|---|
| **1** | **Radix Achyranthis (Root)** | **5-6** |
| **2** | **Fructus Crataegi (Fruit)** | **5-9** |
| **3** | **Radix Scutellariae (Root)** | **4-7** |
| **4** | **Radix Lamiophlomidis (Root)** | **4-8** |
| **5** | **Frutus Lycii (Fruit)** | **5-8** |
| **6** | **Punica grantum(Fruit)** | **8-10** |
| **7** | **Ziniber officnale (Rhizome)** | **4-8** |
| **8** | **Fructus Ribis manschurici (Fruit)** | **2-9** |
| **9** | **Piper longum (Fruit)** | **8-10** |
| **10** | **Coxtex Magnoliae (Bark)** | **2-9** |

## Claims

1. A herbal composition for treating gastric ulcer, wherein the composition is consisting of 4-10% by wt. of an extract from Radix Scutellariae and 4-11% by wt. of an extract from Radix Lamiophlomidis and optionally comprises 5-8 % by wt. of an extract from Radix Achyranthis, 5-11 % by wt. of an extract from Fructus Crataegi, 5- 9% by wt. of an extract from Fructus Lycii, 8-11 % by wt. of an extract from Punica grantum, 4-9% by wt. of an extract from Ziniber officnale, 2-11 % by wt. of an extract from Fructus Ribis manschurici, 8-12% by wt. of an extract from Piper longue and 2- 11 % by wt. of an extract from Coxtex Magnoliae along with one or more pharmaceutically acceptable additives/carriers.

2. A composition as claimed in claim 1, wherein the extract is an aqueous extract.

3. A composition as claimed in claim 1, wherein the plant part of Radix Scutellariae, Radix Lamiophlomidis and Radix Achyranthis is root.

4. A composition as claimed in claim 1, wherein the plant part of Vitis vinifera, Feronia elephaztum, Fructus Ribis manschurici and Piper longum is fruit.

5. A composition as claimed in claim 1, wherein the plant part of Punica grantum is fruit rind.

6. A composition as claimed in claim 1, the plant part of Ziniber officnale is rhizome.

7. A composition as claimed in claim 1, the plant part of Coxtex Magnoliae is bark.

8. A process for the preparation of the herbal composition claimed in one of claims 1-7, wherein the said process comprises getting all the chinese herbes, adding solvent to extract them, and mixing the extracts with one or more pharmaceutically acceptable additives/carriers.

9. A process as claimed in claim 8, wherein the extract is an aqueous extract.
